# EUROPÄISCHE PATENTANMELDUNG

(11) **EP 3 907 007 A1**
(43) Veröffentlichungstag der Anmeldung: **10.11.2021**
(21) Anmeldenummer: 20173705.3
(22) Anmeldetag: 08.05.2020
(51) Int. Cl.: B01L 3/00, C12M 1/32, C12M 3/06, C12M 1/12, G01N 33/50, G01N 33/483

(54) **MIKROFLUIDISCHE VORRICHTUNG**

(71) Anmelder: Technische Universität Wien, 1040 Wien (AT)
(72) Erfinder: Schobesberger, Silvia, 1150 Wien (AT); Rothbauer, Mario, 2511 Pfaffstätten (AT); Ertl, Peter, 1190 Wien (AT); Bachmann, Barbara Eva Maria, 1100 Wien (AT)
(74) Vertreter: Pföstl, Andreas

(57) **Zusammenfassung**

Die vorliegende Erfindung betrifft eine mikrofluidische Vorrichtung 1, vorzugsweise zum Herstellen einer dreidimensionalen Zellkultur, mit mindestens einer Kammer 2, und einem die Kammer 2 zumindest teilweise durchlaufenden Fluidkanal 3 zum Bereitstellen eines die Kammer 2 vorzugsweise kontinuierlich durchlaufenden Fluidstroms, wobei die Kammer 2 mit einer Ladungsöffnung 4 verbunden und über die Ladungsöffnung 4 bis zu einem Sollfüllstand mit Hydrogel beladbar ist, dadurch gekennzeichnet, dass die Kammer 2 eine Hauptkammer 5 und eine mit der Hauptkammer 5 verbundene Nebenkammer 6 umfasst, wobei die Nebenkammer 6 beim Beladen der Kammer 2 mit Hydrogel bis zum Sollfüllstand zumindest teilweise mit Hydrogel befüllbar ist.

## Beschreibung

### TECHNISCHES GEBIET

Die vorliegende Erfindung betrifft das Gebiet von mikrofluidischen Vorrichtungen.

### HINTERGRUND DER ERFINDUNG

3D-Modelle tierischer, insbesondere menschlicher, Zellen ermöglichen es tierisches bzw. menschliches Gewebe in vitro zu rekonstruieren. Wichtige physiologische Bedingungen wie das Aufbringen mechanischer Kräfte oder Diffusionsgradienten konnten bis jetzt jedoch nur begrenzt simuliert werden, obwohl diese für die Funktionalität von 3D-Zellkonstrukten von großer Bedeutung sind. Zur Lösung dieses Problems wurden 3D-Zellkulturtechniken mit Mikrofluidik kombiniert, was diese 3D-Kultivierungsmethode, bezeichnet als Organ-on-a-Chip, physiologisch relevanter macht.

Der Begriff "Organ-on-a-Chip" wurde 2010 von Donald Ingber geschaffen, der ein Lungenmodell auf einem Mikrochip mit Funktionen auf Organebene veröffentlichte (Huh, D., et al., Science, 2010. 328(5986): p. 1662-8).

Hauptvorteil von Organ-on-a-Chips ist die genaue örtlich aufgelöste Kontrolle über Molekül und Flüssigkeiten, wodurch physiologische Molekülgradienten und Flüsse widergespiegelt werden können. Zusätzlich können durch diese Kontrolle verschiedene Zelltypen kontrolliert gemeinsam kultiviert werden, was zu körperähnlicheren Funktionen von Organoiden oder Vaskularisierung der Mikrochips führt.

Die definierten Kammern und Kanäle von mikrofluidischen Vorrichtungen bieten eine reproduzierbare 3D-Zellkulturoberfläche und führen durch die verkleinerten Dimensionen am Mikrochip zu einem geringeren Verbrauch an Zellen und Nährmedium. Da von Tieren bzw. Menschen stammende Zellen verwendet werden, wird außerdem erwartet, dass Organ-on-a-Chips zur Erzeugung pharmazeutisch relevanterer Daten führen als Tierversuche.

Insbesondere bei der Arzneimittelentwicklung führt die Translation von Tierversuchen auf die menschliche Physiologie während klinischer Studien häufig zur Ablehnung des Medikaments - eine finanzielle Belastung für Unternehmen. Organs-on-a-Chip als Krankheitsmodell können einerseits zur Identifizierung neuer Angriffspunkte zur Krankheitsbekämpfung herangezogen und andererseits zur Bewertung der Toxizität und Wirksamkeit von bestehenden Wirkstoffen verwendet werden. Organ-on-a-Chips stellen eine Zwischenstufe in der Komplexität zwischen herkömmlichen 3D-Zellkulturen und Mausmodellen dar, was mit zunehmender Komplexität eine genauere Wirkstoffentwicklung ermöglicht und wodurch sich die Anzahl der nach klinischen Studien zugelassenen Wirkstoffe erhöhen könnte.

Bis heute wurden viele verschiedene Organe, wie Lunge, Darm, Niere oder Plazenta, aber auch Gefäß- und Lymphmodelle und Herz- und Lebermodelle, auf Chips gezüchtet.

Darüber hinaus eröffnete die Technologie induzierter pluripotenter Stammzellen die Möglichkeit der Entwicklung von Gehirnmodellen auf Basis von Organoiden. Da Stammzellen in jeden Zelltyp differenziert werden können, ist es möglich personalisierte Mikrochips unter Verwendung individueller Stammzellen zu entwickeln.

Die Anwendung von Organ-on-a-Chips bleibt eine Herausforderung, da die 3D-Kultur auf dem Chip alle wichtigen Funktionen eines Organs repräsentieren soll. Derartige Modellsysteme müssen eine hohe Reproduzierbarkeit aufweisen, um als valide Alternative zu Tiermodellen zu gelten. Um Hydrogel-basierte Organ-on-a-Chip Systeme reproduzierbarer zu machen, müssen neben den zellulären Aspekten (Zell-Zell Varianz und Heterogenität) auch die technologischen Rahmenbedingungen für das Werkzeug möglichst benutzerfreundlich gestaltet werden. Diese Benutzerfreundlichkeit ist vor allem bei im akademischen Forschungsumfeld entwickelten Organ-on-a-Chip oft fragwürdig, da diese Mikrochips in der Regel von geschultem wissenschaftlichem Personal entwickelt und bedient werden. Für die Industrialisierung und kommerzielle Vermarktung ist es dadurch notwendig, das Design von im wissenschaftlichen Rahmen entwickelte Organs-on-a-Chip bezüglich der Handhabung zu optimieren, um eine reproduzierbare Verwendung durch ungeschultes Personal gewährleisten zu können.

Herkömmliche Organ-on-a-Chip Vorrichtungen weisen in der Regel Kammern auf, in denen ein Organoid durch Zugabe von entsprechenden Zellen ausgebildet werden kann. Da das in den Kammern gebildete Organoid durch Zellzusammenlagerungen ein geringeres Volumen aufweist als die Kammern, in denen sich das Organoid befindet, bewegen sich die ausgebildeten Organoide in den Kammern je nach Stromungsverhältnissen. D.h. eine Messung (z.B. eine optische Messung) direkt an den Organoiden ist entweder nicht oder nur schwer durchführbar, was zu keinem reproduzierbaren Ergebnis führt. Es ist daher eine Aufgabe der vorliegenden Erfindung Mittel und Verfahren bereitzustellen, die es ermöglichen, Organoide innerhalb von Kammern mikrofluidischer Vorrichtungen an eine definiertem Ort innerhalb der Kammern zu positionieren, um eine möglichst geringe Beweglichkeit der Organoide zu gewährleisten.

Eine weitere Aufgabe der vorliegenden Erfindung besteht darin, Kammern von mikrofluidischen Vorrichtungen, die an einen Fluidkanal angrenzen und mit diesem in Kontakt stehen, bereitzustellen, die es ermöglichen Hydrogel, das als Matrix für die Ausbildung von Organoiden vorteilhaft ist, in die Kammern einzubringen, ohne dass dieses in den Fluidkanal eindringt.

### ZUSAMMENFASSUNG DER ERFINDUNG

Erfindungsgemäß werden diese Aufgaben durch eine mikrofluidische Vorrichtung mit mindestens einer Kammer und einem die Kammer zumindest teilweise durchlaufenden Fluidkanal zum Bereitstellen eines die Kammer vorzugsweise kontinuierlich durchlaufenden Fluidstroms gelöst, wobei die Kammer mit einer Ladungsöffnung verbunden und über die Ladungsöffnung bis zu einem Sollfüllstand mit Hydrogel beladbar ist, wobei die Kammer eine Hauptkammer und eine mit der Hauptkammer verbundene Nebenkammer umfasst, wobei die Nebenkammer beim Beladen der Kammer mit Hydrogel bis zum Sollfüllstand zumindest teilweise mit Hydrogel befüllbar ist.

Die erfindungsgemäße mikrofluidische Vorrichtung vorzugsweise zum Herstellen einer dreidimensionalen Zellkultur, insbesondere eines Organoids, umfasst mindestens eine Kammer, und einen die Kammer zumindest teilweise durchlaufenden Fluidkanal zum Bereitstellen eines die Kammer vorzugsweise kontinuierlich durchlaufenden Fluidstroms. Die Kammer ist mit einer Ladungsöffnung verbunden und über die Ladungsöffnung bis zu einem Sollfüllstand mit Hydrogel beladbar. Die Kammer umfasst zudem eine Hauptkammer und eine mit der Hauptkammer verbundene Nebenkammer, wobei die Nebenkammer beim Beladen der Kammer mit Hydrogel bis zum Sollfüllstand zumindest teilweise mit Hydrogel befüllbar ist.

Die Aufteilung der Kammer in eine Hauptkammer und eine Nebenkammer, in Verbindung damit, dass bei der Befüllung der Hauptkammer mit Hydrogel bis zum Sollfüllstand auch die Nebenkammer zumindest teilweise mit Hydrogel befüllt wird, hat den Vorteil, dass beim Ausbilden der dreidimensionalen Zellkultur sich ein Teil der Zellkultur und vor allem ein Teil des sich über die Zeit verdichtendes Hydrogels in der Nebenkammer bildet. Mittels dieses in der Nebenkammer gelegenen Teils wird die gesamte Zellkultur in der Kammer fixiert, wodurch vermieden wird, dass die Zellkultur in der Kammer verrutscht. Hierdurch wird die Reproduzierbarkeit von an der Zellkultur durchgeführten Untersuchungen und Experimenten mit verschiedenen Zellkulturen, welche in mehreren erfindungsgemäßen Vorrichtungen hergestellt wurden, verbessert.

Ein weiterer Aspekt der vorliegenden Erfindung betrifft ein einen Träger umfassend mindestens eine mikrofluidische Vorrichtung gemäß der vorliegenden Erfindung.

Die mikrofluidische Vorrichtung gemäß der vorliegenden Erfindung kann auf einem festen Träger aufgebracht werden. Um möglichst viele Untersuchungen gleichzeitig durchführen zu können, ist es besonders bevorzugt, dass der Träger zwei oder mehr der erfindungsgemäßen Vorrichtungen umfasst.

Ein noch weiterer Aspekt der vorliegenden Erfindung betrifft ein Verfahren zum Herstellen einer dreidimensionalen Zellkultur oder eines Organoids mit einer erfindungsgemäßen mikrofluidischen Vorrichtung, umfassend einen Schritt (A) des Beladens einer Kammer der Vorrichtung durch eine Ladungsöffnung mit Hydrogel bis zu einem Sollfüllstand.

Mit der erfindungsgemäßen Vorrichtung können dreidimensionale Zellkulturen bzw. Organoide hergestellt werden, in dem entsprechende Zellen in die Vorrichtung eingebracht werden. Um die Stabilität der Zellkulturen bzw. der Organoide zu gewährleisten wird als Matrix Hydrogel verwendet.

Ein weiterer Aspekt der vorliegenden betrifft Verfahren zur Bestimmung des Einflusses einer chemischen Verbindung und/oder mindestens eines physikalischen Parameters auf Zellen einer dreidimensionalen Zellkultur in einer mikrofluidischen erfindungsgemäßen Vorrichtung umfassend die Schritte:
a) Feststellen eines Erstzustandes der Zellen,
b1) Inkontaktbringen der Zellen mit einer chemischen Verbindung und/oder
b2) Änderung eines physikalischen Parameters innerhalb der mikrofluidischen Vorrichtung,
c) Feststellen eines Zweitzustandes der Zellen und
d) Bestimmen des Einflusses der chemischen Verbindung und/oder des mindestens einen physikalischen Parameters auf die Zellen durch Ermittlung einer Differenz zwischen dem Erstzustand und Zweitzustand der Zellen.

Die Vorrichtung der vorliegenden Erfindung kann zur Untersuchung des Einflusses einer chemischen Verbindung und/oder mindestens eines physikalischen Parameters auf Zellen, insbesondere auf einer Zellkultur bzw. auf einem Organoid, die bzw. das sich in der Kammer der erfindungsgemäßen Vorrichtung befindet, eingesetzt werden. Vor allem durch die Fixierung der Zellkultur bzw. des Organoids innerhalb der Kammer ist es nun möglich, reproduzierbare Daten zu generieren, da ein möglicher Messpunkt an der Zellkultur bzw. am Organoid in den Kammern konstant ist. Zudem können über den Fluidkanal Medien und Lösungen reproduzierbar zu den Zellen in den Kammern gebracht werden.

### KURZBESCHREIBUNG DER FIGUREN

Figur 1 zeigt eine erfindungsgemäße mikrofluidische Vorrichtung in einer Aufsicht.
Figur 2 zeigt eine Seitenansicht der mikrofluidischen Vorrichtung aus Figur 1.
Figur 3 zeigt eine Aufsicht auf einen Träger mit zwölf erfindungsgemäßen mikrofluidischen Vorrichtungen.
Figur 4 zeigt eine Seitenansicht des in Figur 3 dargestellten Trägers.
Figur 5a, Figur 5b und Figur 5c zeigen die in Figur 2 dargestellte mikrofluidische Vorrichtung während der Beladung der Vorrichtung mit Hydrogel.
Figur 6a, Figur 6b und Figur 6c zeigen eine Draufsicht auf die mikrofluidische Vorrichtung während der Beladung mit Hydrogel.
Figur 7 zeigt eine Seitenansicht der mikrofluidischen Vorrichtung nach der Beladung mit Hydrogel mit einem Fluidkanal.

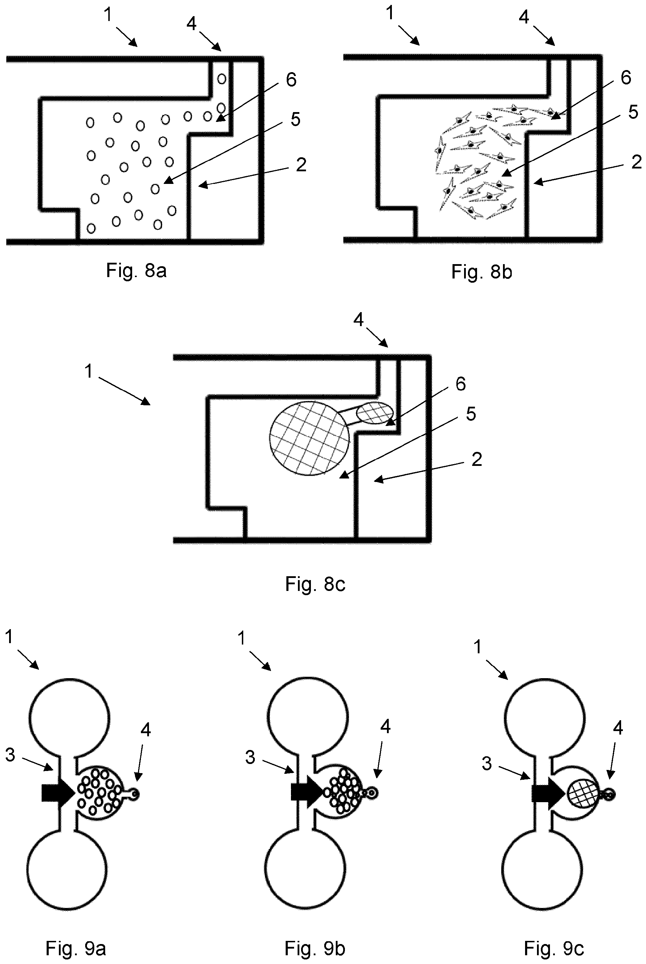

Figur 8a Figur 8b und Figur 8c zeigen eine Seitenansicht der erfindungsgemäßen mikrofluidischen Vorrichtung während der Bildung einer dreidimensionalen Zellkultur.
Figur 9a, Figur 9b und Figur 9c zeigen die mikrofluidische Vorrichtung aus Figur 8a, Figur 8b und Figur 8c in einer Seitenansicht.
Figur 10 zeigt eine Draufsicht auf eine erfindungsgemäße mikrofluidische Vorrichtung während primäre humane synovial Fibroblasten das Hydrogel remodellieren, um im Laufe von vierzehn Tagen zu einer Mikromasse mit physiologischen Strukturen zu werden.
Figur 11 zeigt eine Änderung der Mikromassestruktur von primären humanen Synovialfibroblasten eingebettet in Hydrogel.
Figur 12 zeigt einen Verlauf der Mikromassekondensierung von primären humanen Synovialfibroblasten eingebettet in Hydrogel bei verschiedenen Zellkammerhöhen.
Figur 13 zeigt eine Draufsicht auf einen Träger mit acht erfindungsgemäßen mikrofluidischen Vorrichtungen für eine Ko-Kultur.
Figur 14A und Figur 14B zeigt die Kondensierung von Mikromassen in der erfindungsgemäßen Vorrichtung abhängig von der Passage der humanen synovialen Fibroblasten.
Figur 15 zeigt die Abnahme von Chondrozytenklumpen über die Zeit mit verschiedenen Medien.

### BESCHREIBUNG DER AUSFÜHRUNGSFORMEN

Die erfindungsgemäße Vorrichtung, die nach bekannten Verfahren aus dem Stand der Technik herstellbar ist (siehe beispielsweise Rothbauer M et al. (Lab Chip, 2018, 18, 249-270); Rothbauer M et al. (Biotech Adv, 2015, 33, 948-961); Rothbauer M et al. (Curr Opin Biotech, 2019, 55, 81-86)), eignet sich besonders gut zur Herstellung von dreidimensionalen Zellkulturen und Organoiden und zur Simulation von in vivo ähnlichen Zuständen. Vor allem die Möglichkeit die Zellkulturen bzw. die Organoide mit einem Fluid zu umströmen, ist mit der erfindungsgemäßen Vorrichtung besonders vorteilhaft.

Dreidimensionale Zellkulturen haben den Vorteil, dass die darin befindlichen Zellen, ähnlich wie Organe im Körper eines Menschen oder Tieres, eine räumliche Orientierung einnehmen. Derartige Zellkulturen bestehen aus mehreren Zellschichten, woraus sich deren Dreidimensionalität ergibt. Um die Formgebung zu erleichtern, ist es von Vorteil, zu den Zellen beispielsweise Gerüstproteine/Hydrogele wie etwa Kollagen oder Matrigel dazuzugeben. Es hat sich gezeigt, dass in einer dreidimensionalen Umgebung viele Zelllinien Sphäroide ausbilden.

Auf vielen Forschungsgebieten wird vermehrt auf dreidimensionale Zellkulturen zurückgegriffen, um bestimmte Untersuchungen in einer der Natur ähnlichen bzw. nachempfundenen Umgebung durchführen zu können. Beispielhaft wird auf die pharmazeutische Forschung verwiesen, die - um die Anzahl kostspieliger Tierversuche in der Anfangsphase von Forschungsprojekten zu reduzieren - auf dreidimensionale Zellkulturen zurückgreift. Da dreidimensionale Zellkulturen organähnliches Verhalten aufweisen kann, können aussagekräftige Ergebnisse über Wirkungsweise eines Wirkstoffs erzielt werden, welche sogar Rückschlüsse hinsichtlich Pharmakokinetik und Pharmakodynamik zulassen.

Organoide sind in der Regel nur wenige Millimeter große, organähnliche Mikrostrukturen, die mit der erfindungsgemäßen Vorrichtung hergestellt werden können. Üblicherweise werden Organoide aus Gewebezellen, embryonalen Stammzellen oder induzierten pluripotenten Stammzellen gezüchtet. Trotz der Tatsache, dass kein Stroma und keinerlei Gefäße aufweisen, zeigen diese dennoch physiologisch relevante, organähnliche Eigenschaften. Derzeit sind Organoide für Herz, Magen, Darm, Niere und Gehirn bekannt.

Erfindungsgemäß ist die Kammer der erfindungsgemäßen Vorrichtung bis zu einem "Sollfüllstand" beladbar. "Sollfüllstand", wie hier verwendet, bedeutet, dass in die Kammer so viel Hydrogel eingefüllt werden kann, bis deren Hauptkammer und deren Nebenkammer mit Hydrogel befüllt ist. Die Nebenkammer muss jedenfalls auch Hydrogel umfassen, um den Ankerpunkt der sich ausbildenden dreidimensionalen Zellkultur bzw. des Organoids auszubilden.

Vorzugsweise sind die Hauptkammer und die Nebenkammer durch einen Verbindungskanal verbunden, wobei der Verbindungskanal einen kleineren Querschnitt als die Hauptkammer und die Nebenkammer aufweist. Hierdurch wird der Vorteil erreicht, dass die Zellkultur durch die Querschnittsverengung zwischen der Hauptkammer und der Nebenkammer noch stärker fixiert wird, und die Position der Zellkultur genau festgelegt wird.

Durch das Vorsehen einer derartigen Querschnittsverengung zwischen der Hauptkammer und der Nebenkammer wird noch effizienter und besser gewährleistet, dass bei der Ausbildung der Zellaggregate bzw. der Organoide in der Hauptkammer sich diese kontrolliert Richtung Ladungsöffnung zurückziehen. Es wird gewährleistet, dass dieser Prozess kontrolliert und stets gleich erfolgt. Änderungen der dreidimensionale Zellaggregate bzw. Organoide können mikroskopisch verfolgt werden und durch die definierte Position der dreidimensionalen Zellaggregate/-kulturen bzw. Organoide können Mikroskope, beispielsweise fixiert und automatisiert werden.

Gemäß einer bevorzugten Ausführungsform der erfindungsgemäßen Vorrichtung durchläuft der Fluidkanal die Hauptkammer zumindest teilweise, und die Hauptkammer weist einen ersten Kammerboden auf. Der Fluidkanal umfasst zudem einen, beabstandet von dem ersten Kammerboden in der Hauptkammer angeordneten Fluideinlass, sowie einen beabstandet von dem ersten Kammerboden in der Hauptkammer angeordneten Fluidauslass. Hierdurch wird der Vorteil erreicht, dass eine Stufe zwischen der Hauptkammer und dem Fluideinlass beziehungsweise dem Fluidauslass gebildet wird, welche verhindert, dass Hydrogel beim Beladen der Kammer in den Fluideinlass beziehungsweise den Fluidauslass strömt.

Vorzugsweise weist die Hauptkammer einen ersten Kammerboden, und die Nebenkammer einen zweiten Kammerboden auf, wobei der erste Kammerboden auf einem niedrigeren Höhenniveau als der zweite Kammerboden ausgebildet ist. Hierdurch wird der Vorteil erreicht, dass sich der Großteil des Hydrogels in der Hauptkammer ansammelt, und die Nebenkammer eine Verankerung für eine zum Großteil in der Hauptkammer hergestellte beziehungsweise gezüchtete Zellkultur bietet.

Zweckmäßig weist der Fluidkanal einen Fluidkanalboden auf, wobei der Fluidkanalboden auf einem höheren Höhenniveau als der erste Kammerboden ausgebildet ist. Hierdurch wird verhindert, dass Hydrogel beim Beladen der Kammer und den Fluidkanal strömt.

Gemäß der bevorzugten Ausführungsform der erfindungsgemäßen Vorrichtung ist der Fluidkanalboden auf einem niedrigeren Höhenniveau als der zweite Kammerboden ausgebildet. Hierdurch wird in der Hauptkammer ein Sammelbecken für Hydrogel gebildet, in welchem die Zellkultur heranwachsen kann. Durch den Höhenunterschied zwischen Fluidkanalboden und den ersten Kammerboden wird gewährleistet, dass in die Kammer eingefülltes Hydrogel nicht in den Fluidkanal übertritt solange das in die Kammer eingefüllte Hydrogelvolumen nicht größer ist, als das Kammervolumen. Vorzugsweise ist das eingefüllte Hydrogelvolumen mindestens 5%, noch mehr bevorzugt mindestens 10%, noch mehr bevorzugt mindestens 20%, noch mehr bevorzugt mindestens 30%, geringer als das Kammervolumen.

Gemäß einer bevorzugten Ausführungsform der vorliegenden Erfindung beträgt Höhenunterschied zwischen Fluidkanalboden und dem ersten Kammerboden mindestens 0,1 mm, vorzugsweise mindestens 0,2 mm, noch mehr bevorzugt mindestens 0,25 mm, noch mehr bevorzugt mindestens 0,4 mm.

Gemäß einer besonders bevorzugten Ausführungsform der vorliegenden Erfindung beträgt das Verhältnis des Höhenunterschieds zwischen Fluidkanalboden und dem ersten Kammerboden und der Höhe der Hauptkammer 1:5 zu 1:15, vorzugsweise 1:5 zu 1:12, noch mehr bevorzugt 1:5 zu 1:10, noch mehr bevorzugt 1:6 zu 1:10, noch mehr bevorzugt 1:7 zu 1:9, insbesondere 1:8. Es hat sich gezeigt, dass bei derartigen Höhenverhältnissen das in die Hauptkammer eingebrachte Hydrogel durch eine Fluidstrom im Fluidkanal nicht aus der Hauptkammer herausgeschwemmt wird.

Gemäß einer weiteren bevorzugten Ausführungsform der vorliegenden Erfindung erstreckt sich die Höhe des Fluidkanals vom Fluidkanalboden bis zur Decke der Hauptkammer und steht mit dieser vorzugsweise über die gesamte Höhe in Kontakt.

Vorzugsweise weist die die Nebenkammer ein kleineres Volumen als die Hauptkammer auf. Hierdurch wird ein größerer Masseanteil der Zellkultur in der Hauptkammer gebildet, und die Nebenkammer zur Verankerung der Zellkultur verwendet. Hierdurch kann der in der Hauptkammer verorteten Teil der Zellkultur für Experimente und Untersuchungen genutzt werden.

Gemäß der bevorzugten Ausführungsform ist die Ladungsöffnung mit der Nebenkammer verbunden. Hierdurch wird gewährleistet, dass das Hydrogel erst die Nebenkammer befüllt, bevor es in die Hauptkammer übertritt. Hierdurch wird der Vorteil erreicht, dass sichergestellt wird, dass die Nebenkammer beim Befüllen der Hauptkammer ebenfalls mit Hydrogel befüllt wird.

Vorzugsweise ist die Nebenkammer an einer, dem Fluidkanal gegenüberliegenden Seit der Hauptkammer mit der Hauptkammer verbunden. Hierdurch wird sichergestellt, dass das Hydrogel beim befüllen der Kammer nicht unmittelbar von der Ladungsöffnung über die Nebenhammer in den Fluidkanal abfließt.

Gemäß einer bevorzugten Ausführungsform der vorliegenden Erfindung ist in der Hauptkammer eine den Fluidkanal begrenzende semipermeable Membran, vorzugsweise eine Silikonmembran, angeordnet. Die semipermeable Membran kann ein zusätzliches Hindernis gegen ein Ausspülen der in der Hauptkammer angeordneten Zellkultur/Organoid bzw. des Hydrogels sein.

Gemäß einer Ausführungsvariante der erfindungsgemäßen Vorrichtung umfasst die Vorrichtung eine die Ladungsöffnung mit der Kammer verbindende Leitung. Hierdurch wird der Vorteil erreicht, dass das Hydrogel auf reproduzierbare Weise in die Kammer eingefüllt werden kann, wodurch eine reproduzierbare Verteilung des Hydrogels in der Kammer erreicht wird.

Vorzugsweise weist die Hauptkammer eine Raumdiagonale auf, welche 2 bis 20 mm, vorzugsweise 2 bis 15 mm, noch mehr bevorzugt 3 bis 10 mm, noch mehr bevorzugt 4 bis 6 mm, beträgt.

Zweckmäßig weist des Weiteren der Fluidkanalboden einen Abstand von 0,1 bis 10 mm, vorzugsweise von 0,2 bis 5 mm, noch mehr bevorzugt von 0,2 bis 2 mm, noch mehr bevorzugt von 0,2 bis 1 mm, noch mehr bevorzugt von 0,2 bis 0,4 mm, vom ersten Kammerboden auf.

Gemäß der bevorzugten Ausführungsform umfasst die Kammer, die Hauptkammer, die Nebenkammer und/oder der Fluidkanal eine anwuchsverhindernde Oberflächenbeschichtung umfasst, wobei diese vorzugsweise ausgewählt ist aus der Gruppe bestehend aus einem Polyethylenglykol (PEG)-basierendem Polymer, einem Polysaccharid, insbesondere Agarose, einem Polyhydroxypolymer, insbesondere Poly(2-Hydroxyethylmethacrylat) (poly-HEMA), 2-Methacryloyloxtethylphosphorylcholin (MPC), Dextran oder Hydroxyethylcellulose (HEC), einem natürlichen Polymer, insbesondere einem S-Schichtprotein und Kombinationen davon. Hierdurch wird ein Bewuchs dieser Komponenten durch darin befindlichen Zellen verhindert, so dass es zu keiner Biofilm-Bildung kommt.

Der erfindungsgemäße Träger umfasst mindestens eine erfindungsgemäße mikrofluidische Vorrichtung. Hierdurch wird der Vorteil erreicht, dass eine oder mehrere erfindungsgemäße Vorrichtungen gemeinsam auf einem Träger hergestellt beziehungsweise vorgesehen werden können.

Bevorzugte Ausführungsformen sowie alternative Ausführungsvarianten der erfindungsgemäßen Vorrichtung, des erfindungsgemäßen Trägers und des erfindungsgenmäßen Verfahrens werden in weiterer Folge anhand der Figuren näher erläutert.

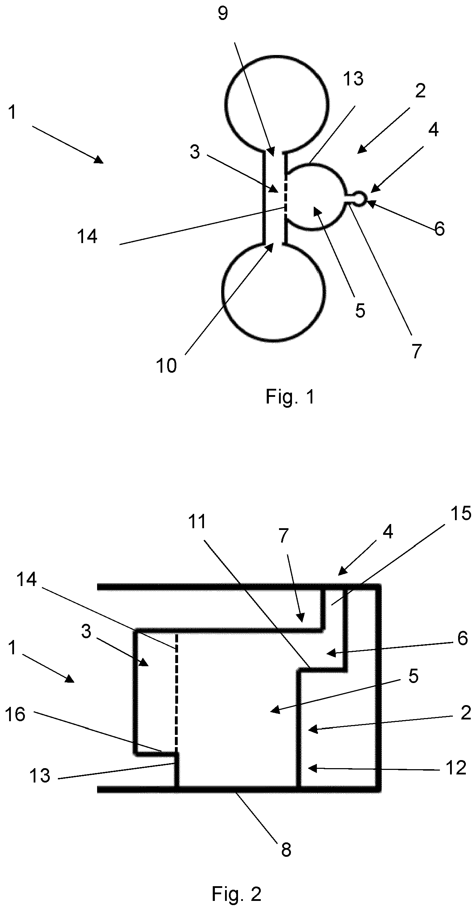

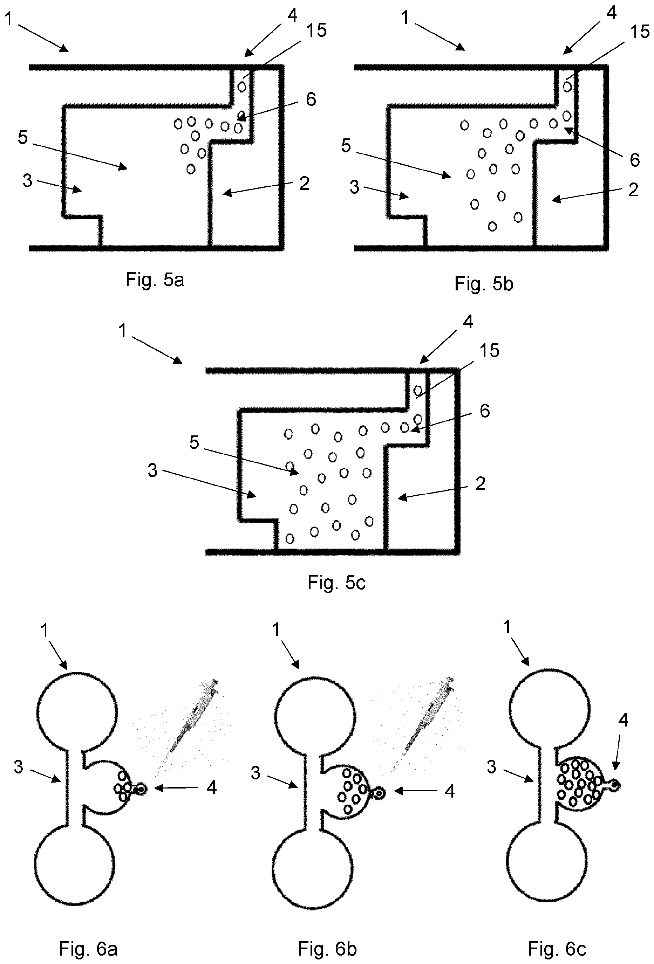

Figur 1 zeigt eine erfindungsgemäße mikrofluidische Vorrichtung 1 vorzugsweise zum Herstellen einer dreidimensionalen Zellkultur in einer Draufsicht mit mindestens einer Kammer 2 und einem die Kammer 2 zumindest teilweise durchlaufenden Fluidkanal 3. In der in Figur 1 dargestellten bevorzugten Ausführungsform der erfindungsgemäßen Vorrichtung 1 weist die Vorrichtung eine Kammer 2 auf, welche von dem Fluidkanal in einem Randbereich der Kammer 2 durchlaufen wird. Der Fluidkanal 3 ist dazu ausgebildet einen die Kammer 2 vorzugsweise kontinuierlich durchlaufenden Fluidstrom bereitzustellen. Die Kammer 2 ist mit einer Ladungsöffnung 4 verbunden und über die Ladungsöffnung 4 bis zu einem Sollfüllstand mit Hydrogel beladbar. Die Beladung mit Hydrogel ist in Figur 5a 5b und 5c dargestellt, und wird weiter unten behandelt. Die Kammer 2 weist eine Hauptkammer 5 und eine mit der Hauptkammer 5 verbundene Nebenkammer 6 auf. Die Hauptkammer 5 und die Nebenkammer 6 sind in Figur 1 und in Figur 2 ersichtlich, wobei Figur 2 eine Seitenansicht der in Figur 1 dargestellten mikrofluidischen Vorrichtung 1 darstellt. Die Nebenkammer 6 ist beim Beladen der Kammer 2 mit Hydrogel bis zum Sollfüllstand zumindest teilweise mit Hydrogel befüllbar. Durch die Aufteilung der Kammer 2 in die Hauptkammer 5 und die Nebenkammer 6, in Verbindung damit, dass bei der Befüllung der Hauptkammer 5 mit Hydrogel bis zum Sollfüllstand auch die Nebenkammer 6 zumindest teilweise mit Hydrogel befüllt wird, wird der Vorteil erreicht, dass beim Ausbilden der dreidimensionalen Zellkultur sich ein Teil der Zellkultur in der Nebenkammer 6 bildet. Mittels dieses in der Nebenkammer 6 gelegenen Teils wird die gesamte Zellkultur in der Kammer 2 fixiert, wodurch vermieden wird, dass die Zellkultur in der Kammer 2 verrutscht. Hierdurch wird die Reproduzierbarkeit von an der Zellkultur durchgeführten Untersuchungen und Experimenten mit verschiedenen Zellkulturen, welche in mehreren erfindungsgemäßen Vorrichtungen 1 hergestellt wurden, verbessert.

Wie in Figur 1 und Figur 2 ersichtlich sind die Hauptkammer 5 und die Nebenkammer 6 gemäß der bevorzugten Ausführungsform der erfindungsgemäßen Vorrichtung 1 durch einen Verbindungskanal 7 verbunden, wobei der Verbindungskanal 7 einen kleineren Querschnitt als die Hauptkammer 5 und die Nebenkammer 6 aufweist. Hierdurch wird der Vorteil erreicht, dass die Zellkultur durch die Querschnittsverengung zwischen der Hauptkammer 5 und der Nebenkammer 6 noch stärker fixiert wird, und die Position der Zellkultur genau festgelegt wird.

Wie in Figur 2 ersichtlich, durchläuft gemäß der bevorzugten Ausführungsform der erfindungsgemäßen Vorrichtung 1 der Fluidkanal 3 die Hauptkammer 5 zumindest teilweise, und die Hauptkammer 5 weist einen ersten Kammerboden 8 auf. Der Fluidkanal 3 umfasst zudem einen, beabstandet von dem ersten Kammerboden 8 in der Hauptkammer 5 angeordneten Fluideinlass 9, sowie einen beabstandet von dem ersten Kammerboden 8 in der Hauptkammer 5 angeordneten Fluidauslass 10. Die Anordnung von Fluideinlass 9 und Fluidauslass 10 ist in Figur 1 ersichtlich. Hierdurch wird der Vorteil erreicht, dass eine Stufe zwischen der Hauptkammer 5 und dem Fluideinlass 9 beziehungsweise dem Fluidauslass 10 gebildet wird, welche verhindert, dass Hydrogel beim Beladen der Kammer 2 in den Fluideinlass 9 beziehungsweise den Fluidauslass 10 strömt.

Vorzugsweise weist die Hauptkammer 5, wie in Figur 2 ersichtlich, den ersten Kammerboden 8, und die Nebenkammer 6 einen zweiten Kammerboden 11 auf. Der erste Kammerboden 8 ist vorzugsweise auf einem niedrigeren Höhenniveau als der zweite Kammerboden 11 ausgebildet. Hierdurch wird der Vorteil erreicht, dass sich der Großteil des Hydrogels in der Hauptkammer 5 ansammelt, und die Nebenkammer 6 eine Verankerung für eine zum Großteil in der Hauptkammer 5 hergestellte beziehungsweise gezüchtete Zellkultur bietet.

Zweckmäßig weist der Fluidkanal 3 einen in Figur 2 dargestellten Fluidkanalboden 16 auf, wobei der Fluidkanalboden 16 auf einem höheren Höhenniveau als der erste Kammerboden 8 ausgebildet ist. Hierdurch wird verhindert, dass Hydrogel beim Beladen der Kammer 2 in den Fluidkanal 3 strömt.

Zudem ist in Figur 2 ersichtlich, dass gemäß der bevorzugten Ausführungsform der erfindungsgemäßen Vorrichtung 1 der Fluidkanalboden 16 auf einem niedrigeren Höhenniveau als der zweite Kammerboden 11 ausgebildet ist. Hierdurch wird in der Hauptkammer 5 ein Sammelbecken für Hydrogel gebildet, in welchem die Zellkultur heranwachsen kann.

Zudem weist die die Nebenkammer 6 gemäß der in den Figuren dargestellten bevorzugten Ausführungsform ein kleineres Volumen als die Hauptkammer 5 auf. Hierdurch wird ein größerer Masseanteil der Zellkultur in der Hauptkammer 5 gebildet, und die Nebenkammer 6 zur Verankerung der Zellkultur verwendet. Hierdurch kann der in der Hauptkammer 5 verortete Teil der Zellkultur für Experimente und Untersuchungen genutzt werden.

Zudem ist in dieser Ausführungsform die Ladungsöffnung 14 mit der Nebenkammer 6, wie in Figur 2 dargestellt, verbunden. Hierdurch wird gewährleistet, dass das Hydrogel erst die Nebenkammer 6 befüllt, bevor es in die Hauptkammer 5 übertritt. Hierdurch wird der Vorteil erreicht, dass sichergestellt wird, dass die Nebenkammer 6 beim Befüllen der Hauptkammer 5 ebenfalls mit Hydrogel befüllt wird.

Vorzugsweise ist die Nebenkammer 6 an einer, dem Fluidkanal gegenüberliegenden Seite 12 der Hauptkammer 5 mit der Hauptkammer 5 verbunden. Hierdurch wird sichergestellt, dass das Hydrogel beim befüllen der Kammer 2 nicht unmittelbar von der Ladungsöffnung 4 über die Nebenkammer 6 in den Fluidkanal 3 abfließt.

Zweckmäßig ist in der Hauptkammer 5 eine den Fluidkanal 3 begrenzende semipermeable Membran 14 angeordnet. Die Silikonmembran 14 ist in Figur 2 mit einer strichlierten Linie dargestellt. Die semipermeable Membran 14 bildet ein Hindernis gegen ein Ausspülen der in der Hauptkammer 5 angeordneten Zellkultur beziehungsweise des Hydrogels durch den Fluidstrom im Fluidkanal 3.

Gemäß einer Ausführungsvariante der erfindungsgemäßen Vorrichtung 1 umfasst die Vorrichtung 1 eine die Ladungsöffnung 4 mit der Kammer 2 verbindende Leitung 15. Dies ist in Figur 2 ersichtlich. Hierdurch wird der Vorteil erreicht, dass das Hydrogel auf reproduzierbare Weise in die Kammer 2 eingefüllt werden kann, wodurch eine reproduzierbare Verteilung des Hydrogels in der Kammer 2 erreicht wird.

Vorzugsweise weist die Hauptkammer 5 eine Raumdiagonale auf, welche 2 bis 20 mm, vorzugsweise 2 bis 15 mm, noch mehr bevorzugt 3 bis 10 mm, noch mehr bevorzugt 4 bis 6 mm, beträgt.

Zweckmäßig weist des Weiteren der Fluidkanalboden 16 einen Abstand von 0,1 bis 10 mm, vorzugsweise von 0,2 bis 5 mm, noch mehr bevorzugt von 0,2 bis 2 mm, noch mehr bevorzugt von 0,2 bis 1 mm, noch mehr bevorzugt von 0,2 bis 0,4 mm, vom ersten Kammerboden 8 auf.

Gemäß der bevorzugten Ausführungsform umfasst die Kammer 2, die Hauptkammer 5, die Nebenkammer 6 und/oder der Fluidkanal 3 eine in den Figuren nicht ersichtliche anwuchsverhindernde Oberflächenbeschichtung umfasst, wobei diese vorzugsweise ausgewählt ist aus der Gruppe bestehend aus einem Polyethylenglykol (PEG)-basierendem Polymer, einem Polysaccharid, insbesondere Agarose, einem Polyhydroxypolymer, insbesondere Poly(2-Hydroxyethylmethacrylat) (poly-HEMA), 2-Methacryloyloxtethylphosphorylcholin (MPC), Dextran oder Hydroxyethylcellulose (HEC), einem natürlichen Polymer, insbesondere einem S-Schichtprotein und Kombinationen davon und/oder ein anwuchsverhinderndes Material basierend auf fluorinierten Silanen oder Phosphatidylcholin-Beschichtungen. Hierdurch wird ein unerwünschter Bewuchs dieser Komponenten durch Zellen verhindert.

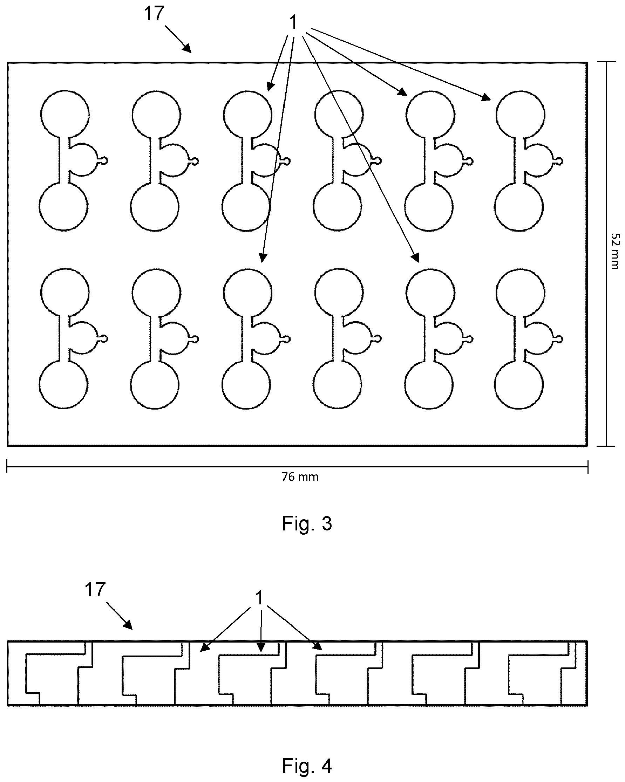

Figur 3 zeigt einen erfindungsgemäßen Träger 17, welcher mindestens eine erfindungsgemäße mikrofluidische Vorrichtung 1 umfasst. Der in Figur 3 dargestellte Träger 17 umfasst 12 erfindungsgemäße mikrofluidische Vorrichtungen 1. Hierdurch wird der Vorteil erreicht, dass eine oder mehrere erfindungsgemäße Vorrichtungen 1 gemeinsam auf einem Träger 17 hergestellt beziehungsweise vorgesehen werden können. Der in Figur 3 beispielhaft dargestellte Träger 17 weist Abmessungen von 76 mm mal 52 mm auf. Figur 4 zeigt den Träger 17 aus Figur 3 in einer Seitenansicht.

In Figur 5a Figur 5b und Figur 5c wird die schrittweise Beladung der erfindungsgemäßen Vorrichtung 1 mit Hydrogel und die Ausbreitung des Hydrogels in der Vorrichtung 1 im Zuge der Beladung dargestellt. Das Hydrogel wird mit kleinen Kugeln symbolisiert. Es breitet sich über die Ladungsöffnung 4 über die Nebenkammer 6 in die Hauptkammer 5 aus.

Die Figur 6a, Figur 6b und Figur 6c zeigt die in den Figuren 5a, 5b und 5c dargestellte Ausbreitung in einer Draufsicht, mit einer Spritze, welche für die Einbringung des Hydrogels in die Ladungsöffnung 4 genutzt wird.

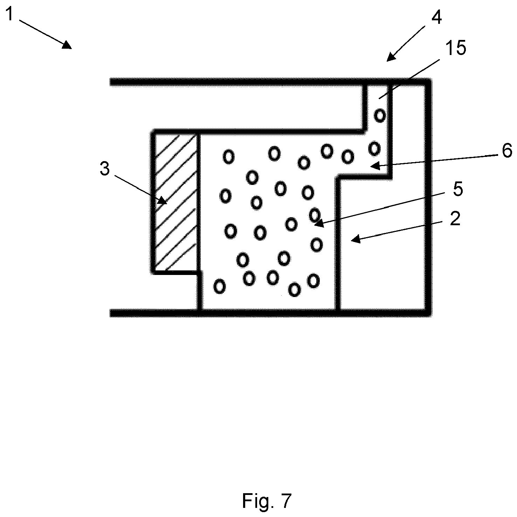

Figur 7 zeigt die erfindungsgemäße Vorrichtung 1 in einem mit Hydrogel beladenen Zustand, wobei der Fluidkanal 3 mit einer schraffierten Fläche gekennzeichnet und frei von Hydrogel ist.

Ein weiterer Aspekt der vorliegenden Erfindung betrifft ein Verfahren zum Herstellen einer dreidimensionalen Zellkultur oder eines Organoids mit einer mikrofluidischen Vorrichtung gemäß der vorliegenden Erfindung, umfassend einen Schritt (A) des Beladens einer Kammer der erfindungsgemäßen Vorrichtung durch eine Ladungsöffnung mit Hydrogel bis zu einem Sollfüllstand.

In einem ersten Schritt (A) des erfindungsgemäßen Verfahrens wird Hydrogel in die Kammer eingebracht. Dabei wird so viel Hydrogel in die Kammer eingefüllt bis es zu einem Rückstau des Hydrogels bis zur Nebenkammer kommt. In einem solchen Fall ist gewährleistet, dass das zusammenziehende Hydrogel bzw. Zell/Hydrogel-Gemisch an der Nebenkammer der Hauptkammer fixiert bleibt.

Gemäß einer bevorzugten Ausführungsform der vorliegenden Erfindung werden Zellen gemeinsam in und/oder vor Schritt (A) durch eine Ladungsöffnung und/oder über einen Fluideinlass (9) in die Kammer (2) eingebracht werden.

Im erfindungsgemäßen Verfahren können die Zellen nach und/oder vor Schritt (A) (Einbringung von Hydrogel in die Kammer der erfindungsgemäßen Vorrichtung) in die Kammer appliziert werden. Alternativ dazu ist es auch möglich die Zellen gemeinsam mit dem Hydrogel in die Kammer einzubringen.

Gemäß einer weiteren bevorzugten Ausführungsform der vorliegenden Erfindung sind die Zellen ausgewählt aus Stammzellen und primären Zellen, vorzugsweise Fibroblasten, Knorpelzellen, Endothelzellen, Epithelzellen, Fettzellen, induziert pluripotente Stammzellen (IPS), Osteoclasten, Osteoblasten oder Osteozyten.

In die erfindungsgemäße Vorrichtung können Zellen, insbesondere tierische oder menschliche Zellen, verschiedenster Herkunft eingebracht werden, sofern diese in der Lage sind, eine dreidimensionale Struktur (wie z.B. ein Organoid) auszubilden. Werden beispielsweise Stammzellen in die Vorrichtung eingebracht, können diese direkt in der Vorrichtung nach bekannten Verfahren ausdifferenziert werden.

Das Kultivierungsmedium, welches durch den Fluidkanal in die Kammer eingebracht wird, wird entsprechend der eingesetzten Zellen ausgewählt. Entsprechende Medien sind dem Fachmann hinreichend bekannt.

Gemäß einer bevorzugten Ausführungsform der vorliegenden Erfindung umfasst oder besteht das Hydrogel aus Matrigel, Fibrin, Kollagen, PEG-basiertes Hydrogel, Gelatine-basiertes Hydrogel, Hyaluronsäure-basiertes Hydrogel, Alginat-basierts Hydrogel, Seide-basiertes Hydrogel und Kombinationen davon.

Das Hydrogel weist vorzugsweise eine Konzentration von 0,1 mg/ml bis 500 mg/ml auf, noch mehr bevorzugt von 0,5 mg/ml bis 500 mg/ml.

Gemäß einer bevorzugten Ausführungsform der vorliegenden Erfindung wird die erfindungsgemäße Vorrichtung nach Schritt (A) für 12 Stunden bis 60 Tage, vorzugsweise für 1 bis 30 Tage, noch mehr bevorzugt für 2 bis 20 Tage, inkubiert.

Um Organoide oder dreidimensionale Zellkulturen herzustellen, ist es von Vorteil, die Vorrichtung umfassend die Zellen und das Hydrogel für eine bestimmte Zeitdauer bei Bedingungen zu inkubieren, die ein Wachstum der Zellen ermöglicht.

Gemäß einer besonders bevorzugten Ausführungsform der vorliegenden Erfindung wird die erfindungsgemäße Vorrichtung bei einer Temperatur von 25 bis 38°C, vorzugsweise von 37 bis 38°C, inkubiert wird.

Die Temperatur, bei der die erfindungsgemäße Vorrichtung inkubiert wird, richtet sich nach den zu kultivierenden Zellen und wird vorzugsweise in einem Bereich von 25 bis 38°C variiert.

Gemäß einer weiteren bevorzugten Ausführungsform der vorliegenden Erfindung wird nach Schritt (A) in einem Schritt (B) die Kammer mit einem durch einen Fluidkanal kontinuierlich oder diskontinuierlich durchlaufenden Fluidstrom versorgt wird, wobei bei einem diskontinuierlichen Fluidstrom das Fluid vorzugsweise verändert.

Während der Kultivierung der Zellen innerhalb der erfindungsgemäßen Vorrichtung kann der Fluidstrom im Fluidkanal kontinuierlich oder diskontinuierlich (d.h. mit Unterbrechungen oder veränderter Flussrate) sein. Zudem ist es möglich die Zusammensetzung des Fluids (z.B. Nährmedium), welches durch den Fluidkanal fließt, über die Zeit bzw. während der Kultivierung der Zellen zu verändern. Dadurch können einerseits natürliche Vorgänge simuliert werden, da sich auch im menschlichen bzw. tierischen Körper die Nährstoffzufuhr bzw. ein Gewebe "umspülender" Fluidstromändert bzw. ändern kann. Die Änderung der Flussgeschwindigkeit und Zusammensetzung des Fluids im Fluidkanal hat zudem den Vorteil, dass der Einfluss von Fluidströmen und Substanzen auf das Zellwachstum oder dem Zustand der Zellen innerhalb der erfindungsgemäßen Vorrichtung untersucht werden kann.

Gemäß einer bevorzugten Ausführungsform der vorliegenden Erfindung wird der CO₂- und/oder O₂-Gehalt und/oder der pH-Wert innerhalb der Vorrichtung und/oder des Fluidstroms kontrolliert wird.

Um das Wachstum der Zellen und deren Zustand innerhalb der erfindungsgemäßen Vorrichtung zu beobachten, ist es von Vorteil bestimmte Parameter, insbesondere die oben genannten Parameter, zu erfassen und zu kontrollieren, um gegebenenfalls entsprechende Maßnahmen zu setzen.

Ein noch weiterer Aspekt der vorliegenden Erfindung betrifft ein Verfahren zur Bestimmung des Einflusses einer chemischen Verbindung und/oder mindestens eines physikalischen Parameters auf Zellen einer dreidimensionalen Zellkultur in einer erfindungsgemäßen mikrofluidischen Vorrichtung umfassend die Schritte:
a) Feststellen eines Erstzustandes der Zellen,
b1) Inkontaktbringen der Zellen mit einer chemischen Verbindung und/oder
b2) Änderung eines physikalischen Parameters innerhalb der mikrofluidischen Vorrichtung,
c) Feststellen eines Zweitzustandes der Zellen und
d) Bestimmen des Einflusses der chemischen Verbindung und/oder des mindestens einen physikalischen Parameters auf die Zellen durch Ermittlung einer Differenz zwischen dem Erstzustand und Zweitzustand der Zellen.

Mit der erfindungsgemäßen Vorrichtung ist es möglich den Einfluss von physikalischen Parametern oder Substanzen bzw. Substanzgemischen auf dreidimensionale Zellkulturen bzw. Organoide. Dabei gilt es zunächst festzustellen, in welchem Zustand sich die Zellen vor Zugabe einer Substanz bzw. einen Substanzmischung bzw. vor Änderung der physikalischen Parameter befinden. Die Feststellung, in welchem Zustand sich die Zellen befinden, kann mit verschiedenen Methoden erfolgen. Es ist einerseits möglich zu bestimmen, ob die Zellkultur bzw. das Organoid bestimmte Substanzen (z.B. Chemokine) ausschüttet. Andererseits können auch rein optische Methoden herangezogen werden, bei denen der optische Zustand der Kultur bzw. des Organoids betrachtet wird. Dieselben Untersuchungen werden nach Schritt b1) und/oder b2) durchgeführt, um einen Zweitzustand festzstellen. Aus der Differenz zwischen Erst- und Zweitzustand lässt sich der Einfluss eines physikalischen Parameters oder einer Substanz bzw. eines Substanzgemisches auf die Zellen ableiten.

Der Einfluss der chemischen Verbindung und/oder des mindestens einen physikalischen Parameters auf die Zellen kann vorzugsweise durch Messung der Viabilität, Morphologie, Sekretion von Substanzen aus den Zellen, optischen Transparenz und/oder Eintrübung oder Kombinationen davon bestimmt werden.

Derartige Verfahren sind beispielsweise in Kratz SRA et al. (Biosensors 2019, 9(3), 110) erwähnt.

Gemäß einer bevorzugten Ausführungsform der vorliegenden Erfindung wird der Einfluss der chemischen Verbindung und/oder des mindestens einen physikalischen Parameters auf die Zellen durch Messung der Ausschüttung von Biomolekülen, insbesondere von Antikörpern, Chemokinen, Zytokinen, Enzymen oder microRNA, bestimmt

Besonders bevorzugt die Mengen bzw. Konzentration von Interleukinen, Matrixmetalloproteinasen, Wachstumsfaktoren wie BMP, EGF; VEGF, FGF, BAFF, usw. bestimmt (siehe z.B. Rothbauer M et al. (Lab Chip, 2018, 18, 249-270)).

Gemäß einer bevorzugten Ausführungsform der vorliegenden Erfindung ist der physikalische Parameter ausgewählt aus der Gruppe bestehend aus Temperatur und pH-Wert.

Die vorliegende Erfindung wird anhand der folgenden Ausführungsformen und Beispiele näher dargelegt, ohne jedoch auf diese beschränkt zu sein.

### AUSFÜHRUNGSFORMEN

1. Mikrofluidische Vorrichtung (1), vorzugsweise zum Herstellen einer dreidimensionalen Zellkultur, mit mindestens einer Kammer (2) und einem die Kammer (2) zumindest teilweise durchlaufenden Fluidkanal (3) zum Bereitstellen eines die Kammer (2) vorzugsweise kontinuierlich durchlaufenden Fluidstroms, wobei die Kammer (2) mit einer Ladungsöffnung (4) verbunden und über die Ladungsöffnung (4) bis zu einem Sollfüllstand mit Hydrogel beladbar ist, dadurch gekennzeichnet, dass die Kammer (2) eine Hauptkammer (5) und eine mit der Hauptkammer (5) verbundene Nebenkammer (6) umfasst, wobei die Nebenkammer (6) beim Beladen der Kammer (2) mit Hydrogel bis zum Sollfüllstand zumindest teilweise mit Hydrogel befüllbar ist.
2. Vorrichtung (1) gemäß Ausführungsform 1, dadurch gekennzeichnet, dass die Hauptkammer (5) und die Nebenkammer (6) durch einen Verbindungskanal (7) verbunden sind, wobei der Verbindungskanal (7) einen kleineren Querschnitt als die Hauptkammer (5) und die Nebenkammer (6) aufweist.
3. Vorrichtung (1) gemäß einer der Ausführungsformen 1 oder 2, dadurch gekennzeichnet, dass der Fluidkanal (3) die Hauptkammer (5) zumindest teilweise durchläuft, und die Hauptkammer (5) einen ersten Kammerboden (8) aufweist, wobei der Fluidkanal (3) einen, beabstandet von dem ersten Kammerboden (8) in der Hauptkammer (5) angeordneten Fluideinlass (9), sowie einen beabstandet von dem ersten Kammerboden (8) in der Hauptkammer (5) angeordneten Fluidauslass (10) umfasst.
4. Vorrichtung (1) gemäß einer der Ausführungsformen 1 bis 3, dadurch gekennzeichnet, dass die Hauptkammer (5) einen ersten Kammerboden (8) aufweist und die Nebenkammer (6) einen zweiten Kammerboden (11) aufweist, wobei der erste Kammerboden (8) auf einem niedrigeren Höhenniveau als der zweite Kammerboden (11) ausgebildet ist.
5. Vorrichtung (1) gemäß Ausführungsform 3 oder 4, dadurch gekennzeichnet, dass der Fluidkanal (3) einen Fluidkanalboden (16) aufweist, wobei der Fluidkanalboden (16) auf einem höheren Höhenniveau als der erste Kammerboden (8) ausgebildet ist.
6. Vorrichtung (1) gemäß Ausführungsform 5, dadurch gekennzeichnet, dass der Fluidkanalboden (16) auf einem niedrigeren Höhenniveau als der zweite Kammerboden (11) ausgebildet ist.
7. Vorrichtung (1) gemäß einer der Ausführungsformen 1 bis 6, dadurch gekennzeichnet, dass die Nebenkammer (6) ein kleineres Volumen als die Hauptkammer (5) aufweist.
8. Vorrichtung (1) gemäß einer der Ausführungsformen 1 bis 7, dadurch gekennzeichnet, dass die Ladungsöffnung (4) mit der Nebenkammer (6) verbunden ist.
9. Vorrichtung (1) gemäß einer der Ausführungsformen 1 bis 8, dadurch gekennzeichnet, dass die Nebenkammer (6) an einer, dem Fluidkanal (3) gegenüberliegenden Seite (12) der Hauptkammer (5) mit der Hauptkammer (5) verbunden ist.
10. Vorrichtung (1) gemäß einer der Ausführungsformen 1 bis 9, dadurch gekennzeichnet, dass der Fluidkanal (3) entlang einer Seitenwand (13) der Hauptkammer (5) verläuft.
11. Vorrichtung (1) gemäß einer der Ausführungsformen 1 bis 10, dadurch gekennzeichnet, dass in der Hauptkammer (5) eine den Fluidkanal (3) begrenzende semipermeable Membran (14), vorzugsweise eine Silikonmembran, angeordnet ist.
12. Vorrichtung (1) gemäß einer der Ausführungsformen 1 bis 11, dadurch gekennzeichnet, dass die Vorrichtung (1) eine die Ladungsöffnung (4) mit der Kammer (2) verbindende Leitung (15) umfasst.
13. Vorrichtung (1) gemäß einer der Ausführungsformen 1 bis 12, dadurch gekennzeichnet, dass die Hauptkammer (5) eine Raumdiagonale aufweist, welche 2 bis 20 mm, vorzugsweise 2 bis 15 mm, noch mehr bevorzugt 3 bis 10 mm, noch mehr bevorzugt 4 bis 6 mm, beträgt.
14. Vorrichtung (1) gemäß einer der Ausführungsformen 5 bis 13, dadurch gekennzeichnet, dass der Fluidkanalboden (16) einen Abstand von 0,1 bis 10 mm, vorzugsweise von 0,2 bis 5 mm, noch mehr bevorzugt von 0,2 bis 2 mm, noch mehr bevorzugt von 0,2 bis 1 mm, noch mehr bevorzugt von 0,2 bis 0,4 mm, vom ersten Kammerboden (8) aufweist.
15. Vorrichtung (1) gemäß einer der Ausführungsformen 1 bis 14, dadurch gekennzeichnet, dass die Kammer (2), die Hauptkammer (5), die Nebenkammer (6) und/oder der Fluidkanal (3) eine anwuchsverhindernde Oberflächenbeschichtung umfasst, wobei diese vorzugsweise ausgewählt ist aus der Gruppe bestehend aus einem Polyethylenglykol (PEG)-basierendem Polymer, einem Polysaccharid, insbesondere Agarose, einem Polyhydroxypolymer, insbesondere Poly(2-Hydroxyethylmethacrylat) (poly-HEMA), 2-Methacryloyloxtethylphosphorylcholin (MPC), Dextran oder Hydroxyethylcellulose (HEC), einem natürlichen Polymer, insbesondere einem S-Schichtprotein und Kombinationen davon.
16. Träger (17) umfassend mindestens eine mikrofluidische Vorrichtung gemäß einem der Ansprüche 1 bis 15.
17. Verfahren zum Herstellen einer dreidimensionalen Zellkultur oder eines Organoids mit einer mikrofluidischen Vorrichtung (1) gemäß einem der Ansprüche 1 bis 15, umfassend einen Schritt (A) des Beladens einer Kammer (2) der Vorrichtung (1) durch eine Ladungsöffnung (4) mit Hydrogel bis zu einem Sollfüllstand.
18. Verfahren gemäß Ausführungsform 17, dadurch gekennzeichnet, dass Zellen gemeinsam in und/oder vor Schritt (A) durch eine Ladungsöffnung (4) und/oder über einen Fluideinlass (9) in die Kammer (2) eingebracht werden.
19. Verfahren gemäß Ausführungsform 18, dadurch gekennzeichnet, dass die Zellen ausgewählt sind aus Stammzellen und primären Zellen, vorzugsweise Fibroblasten, Knorpelzellen, Endothelzellen, Epithelzellen, Fettzellen, induziert pluripotente Stammzellen (IPS), Osteoclasten, Osteoblasten oder Osteozyten.
20. Verfahren gemäß einer der Ausführungsformen 17 bis 19, dadurch gekennzeichnet, dass das Hydrogel Matrigel, Fibrin, Kollagen, PEG-basiertes Hydrogel, Gelatine-basiertes Hydrogel, Hyaluronsäure-basiertes Hydrogel, Alginat-basierts Hydrogel, Seide-basiertes Hydrogel und Kombinationen davon umfasst oder daraus besteht.
21. Verfahren gemäß einer der Ausführungsformen 17 bis 20, dadurch gekennzeichnet, dass die Vorrichtung (1) nach Schritt (A) für 12 Stunden bis 60 Tage, vorzugsweise für 1 bis 30 Tage, noch mehr bevorzugt für 2 bis 20 Tage, inkubiert wird.
22. Verfahren gemäß Ausführungsform 21, dadurch gekennzeichnet, dass die Vorrichtung (1) bei einer Temperatur von 25 bis 38°C, vorzugsweise von 37 bis 38°C, inkubiert wird.
23. Verfahren gemäß einer der Ausführungsformen 17 bis 22, dadurch gekennzeichnet, dass nach Schritt (A) in einem Schritt (B) die Kammer (2) mit einem durch einen Fluidkanal (3) kontinuierlich oder diskontinuierlich durchlaufenden Fluidstrom versorgt wird, wobei bei einem diskontinuierlichen Fluidstrom das Fluid vorzugsweise verändert wird.
24. Verfahren gemäß einer der Ausführungsformen 17 bis 23, dadurch gekennzeichnet, dass der CO₂- und/oder O₂-Gehalt und/oder der pH-Wert innerhalb der Vorrichtung und/oder des Fluidstroms kontrolliert wird.
25. Verfahren zur Bestimmung des Einflusses einer chemischen Verbindung und/oder mindestens eines physikalischen Parameters auf Zellen einer dreidimensionalen Zellkultur in einer mikrofluidischen Vorrichtung gemäß einem der Ansprüche 1 bis 16 umfassend die Schritte:
   a) Feststellen eines Erstzustandes der Zellen,
   b1) Inkontaktbringen der Zellen mit einer chemischen Verbindung und/oder
   b2) Änderung eines physikalischen Parameters innerhalb der mikrofluidischen Vorrichtung,
   c) Feststellen eines Zweitzustandes der Zellen und
   d) Bestimmen des Einflusses der chemischen Verbindung und/oder des mindestens einen physikalischen Parameters auf die Zellen durch Ermittlung einer Differenz zwischen dem Erstzustand und Zweitzustand der Zellen.
26. Verfahren gemäß Ausführungsform 25, dadurch gekennzeichnet, dass der Einfluss der chemischen Verbindung und/oder des mindestens einen physikalischen Parameters auf die Zellen durch Messung der Viabilität, Morphologie, Sekretion von Substanzen aus den Zellen, optischen Transparenz und/oder Eintrübung oder Kombinationen davon bestimmt wird
27. Verfahren gemäß Ausführungsform 25 oder 26, dadurch gekennzeichnet, dass der Einfluss der chemischen Verbindung und/oder des mindestens einen physikalischen Parameters auf die Zellen durch Messung der Ausschüttung von Biomolekülen, insbesondere von Antikörpern, Chemokinen, Zytokinen, Enzymen oder microRNA, bestimmt wird
28. Verfahren nach einer der Ausführungsformen 25 bis 27, dadurch gekennzeichnet, dass der physikalische Parameter ausgewählt ist aus der Gruppe bestehend aus Temperatur und pH-Wert.

### BEISPIELE

### Beispiel 1: Charakterisierung des Kondensationsverhalten von synovialen Fibroblasten

### Methoden

### Chipherstellung

Zur Reinigung der Grundlage des Chips, ein Objektträger (76 x 26 mm) von VWR, wurde zuerst in eine 2% Hellmanex® III Lösung gegeben und 5 Minuten ins Ultraschallbad behandelt. Anschließend wurde die Lösung verworfen und der Vorgang zuerst mit Isopropanol und anschließend mit Wasser wiederholt. Zuletzt wurden die Objektträger mit Druckluft und 1 Stunde bei 80 °C im Ofen getrocknet.

Für die Chipherstellung, mussten zuerst 5 PDMS Schichten (76 x 26 mm) ausgeschnitten werden. Dafür wurde 0.5 mm dicke PDMS Folie in den Schneider CAMM-1 GS-24 von Roland DG eingefügt und die gewünschte Form ausgeschnitten, welche mit einer Pinzette entfernt wurden. Dann wurden die einzelnen Schichten durch Plasmaaktivierung miteinander verbunden, beginnend mit dem Mikroskopslide und der ersten Schicht PDMS Folie. Die Stärke der Verbindung wurde durch Hitzebehandlung im Ofen (80 °C) verstärkt.

Vor der Verwendung des Chips wurden alle Kammern, Kanäle und Reservoirs mit 70% Ethanol gespült. Die Zellkammern wurden zudem mit Lipidure®, einem Antifouling-Mittel, beschichtet. Zuletzt wurde der Chip mit UV-Licht sterilisiert.

### Zellkultur

Synoviale Fibroblasten wurden mit Kulturmedium (DMEM, 10% FBS, 1% Antibiotikum, 1% MEM NEAA) in Kulturflaschen kultiviert (37 °C, 5% CO2) und bei 90% Konfluenz aus der Flasche gelöst. Dafür wurden die Zellen 5 Minuten mit 1X Trypsinlösung bei 37 °C inkubiert und anschließend Medium hinzugefügt, um die Reaktion zu stoppen. Die Zellsuspension wurden mittels Trypanblau gezählt und zentrifugiert (4 °C, 1400 rpm, 5 min). Das Zellpellet wurde anschließend in Medium resuspendiert und die benötigte Menge an Zellen abgetrennt. Nach erneutem Zentrifugieren wurde das Zellpellet mit Matrigel vermischt (3000 Zellen pro µl Matrigel) und die Chips mit 45 µl Zell-/Matrigelsuspension pro Zellkammer befüllt. Nach 40 Minuten Polymerisierung bei 37 °C wurde 3D Kultivierungsmedium hinzugefügt (DMEM, 10% FBS, 1% Antibiotikum, 1% MEM NEAA, 1% ITS, 31.6 µg/ml L-Ascorbic acid 2-phosphate, 2% HEPES). Die Chips wurden bis zu 4 Wochen bei 37 °C und 5% CO2 kultiviert, wobei das Medium zweimal in der Woche getauscht wurde.

### Resultate und Diskussion

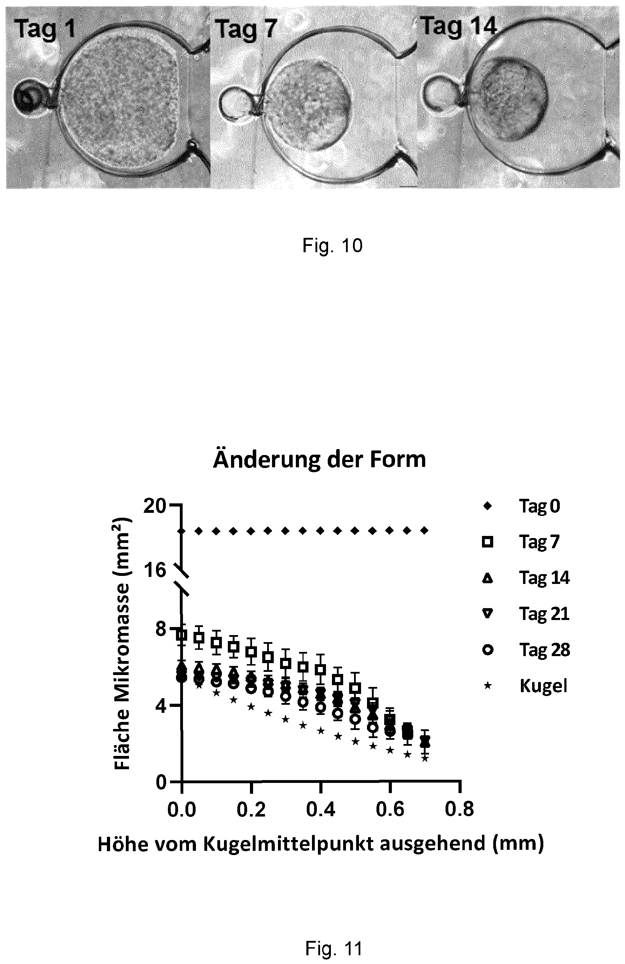

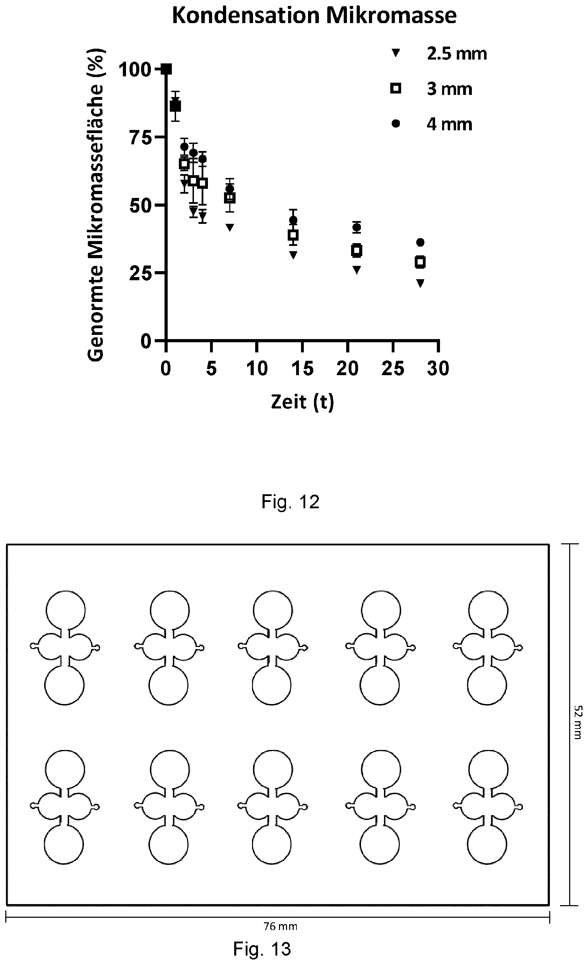

Die synovialen Fibroblasten gehen Zell-Zell-Verbindungen ein und beginnen eigenen extrazelluläre Matrix auszuschütten. Das Matrigel, in dem sich die Zellen anfangs befinden, wird umgebaut und durch die starken Zellverbindungen kondensiert die so genannte Mikromasse über die Zeit (Figur 10). Die Kondensation erfolgt dabei kontrolliert zurück Richtung Ladungsöffnung, da übrige Zellen im Ladungskanal eine miniaturisierte Mikromasse bilden. Diese kann nicht in die eigentliche Zellkammer, da der Einlaufkanal verengt ist. Die miniaturisierte Mikromasse ist mit der großen Mikromasse in der Zellkammer durch Zellen verbunden. Während des Umbaus des Matrigels und der Kondensation entsteht eine Mikromasse ähnlich synovialem Gewebe. Charakteristisch für synoviales Gewebe ist eine dichtere Zellschicht Richtung Medium, sowie ein eher lockeres Gewebe im Inneren der Mikromasse. Durch die Kondensation ändert sich die Struktur, da zu Beginn die Zell-/Matrigelsuspension in einer zylinderartigen Kammer polymerisiert. Durch das Zusammenziehen bildet sich eine kugelartige Mikromasse. Um diese Annahme zu überprüfen wurden Phasenkontrastbilder in regelmäßigen Abständen über die z-Achse von der Mikromasse aufgenommen. Anhand der Schärfeebene konnte die Größe des Querschnitts an verschiedenen Positionen bestimmt werden. Figure 11 zeigt, dass sich die Form nach 14 Tagen nur noch wenig ändert. Ein Vergleich mit einer Kugel zeigt, dass die Kurve jedoch etwas anders verlaufen müsste. Daher ist die Annahme, dass die Mikromasse ellipsoid geformt ist, da der Durchmesser der Kammer 2,5-mal so groß ist, wie die Höhe. Um den Einfluss der Höhe auf die Kondensation abzuwägen, wurden Chips mit unterschiedlich hohen Zellkammer gebaut. Figure 12 zeigt, dass keine wesentlichen Änderungen im Kondensationverhalten zu beobachten sind. Unterschiede beruhen hauptsächlich auf dem Volumensunterschied.

### Beispiel 2: Kondensationsverhalten von synovialen Fibroblasten in Gegenwart von Immunzellen

### Methoden

### Chipherstellung

Zur Reinigung der Grundlage des Chips, ein Objektträger (76 x 26 mm) von VWR, wurde zuerst in eine 2% Hellmanex® III Lösung gegeben und 5 Minuten ins Ultraschallbad behandelt. Anschließend wurde die Lösung verworfen und der Vorgang zuerst mit Isopropanol und anschließend mit Wasser wiederholt. Zuletzt wurden die Objektträger mit Druckluft und 1 Stunde bei 80 °C im Ofen getrocknet.

Für die Chipherstellung, mussten zuerst 5 PDMS Schichten (76 x 26 mm) ausgeschnitten werden. Dafür wurde 0.5 mm dicke PDMS Folie in den Schneider CAMM-1 GS-24 von Roland DG eingefügt und die gewünschte Form ausgeschnitten, welche mit einer Pinzette entfernt wurden. Dann wurden die einzelnen Schichten durch Plasmaaktivierung miteinander verbunden, beginnend mit dem Mikroskopslide und der ersten Schicht PDMS Folie. Die Stärke der Verbindung wurde durch Hitzebehandlung im Ofen (80 °C) verstärkt.

Vor der Verwendung des Chips wurden alle Kammern, Kanäle und Reservoirs mit 70% Ethanol gespült. Die Zellkammern wurden zudem mit Lipidure®, einem Antifouling-Mittel, beschichtet. Zuletzt wurde der Chip mit UV-Licht sterilisiert.

### Zellkultur

Zellsuspensionen von OP-Präparaten wurden aufgetaut und mit Kulturmedium (DMEM, 10% FBS, 1% Antibiotikum, 1% MEM NEAA) in Kulturflaschen kultiviert (37 °C, 5% CO2). Bei 90% Konfluenz wurden die Zellen aus der Flasche gelöst. Dafür wurden die Zellen 5 Minuten mit 1X Trypsinlösung bei 37 °C inkubiert und anschließend Medium hinzugefügt, um die Reaktion zu stoppen. Die Zellsuspension wurden mittels Trypanblau gezählt und zentrifugiert (4 °C, 1400 rpm, 5 min). Die Zellen wurden in Medium resuspendiert und ein Teil wurde subkultiviert, sowie ein anderer Teil mit der benötigten Menge an Zellen für die Chipkulturen abgetrennt. Nach erneutem Zentrifugieren wurde das Zellpellet mit Matrigel vermischt (3000 Zellen pro µl Matrigel) und die Chips mit 45 µl Zell-/Matrigelsuspension pro Zellkammer befüllt. Nach 40 Minuten Polymerisierung bei 37 °C wurde 3D Kultivierungsmedium hinzugefügt (DMEM, 10% FBS, 1% Antibiotikum, 1% MEM NEAA, 1% ITS, 31.6 µg/ml L-Ascorbic acid 2-phosphate, 2% HEPES). Die Chips wurden bis zu 4 Wochen bei 37 °C und 5% CO2 kultiviert, wobei Medium zweimal in der Woche getauscht wurde.

### Resultate und Diskussion

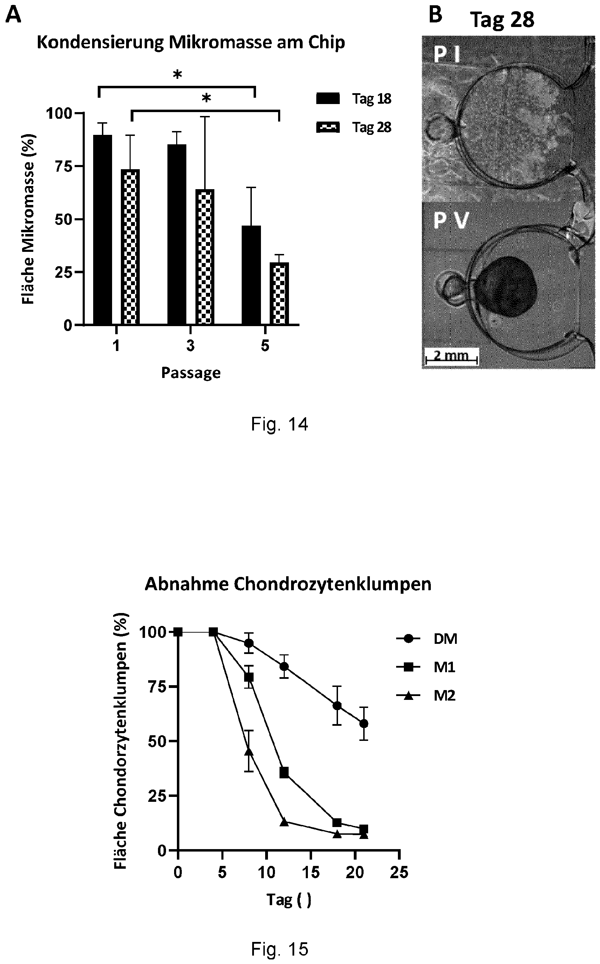

In frühen Passagen sind noch wesentlich mehr Immunzellen vorhanden, welche einen Einfluss auf die Mikromassebildung haben, da die Umgebung ein entzündetes Gelenk nachahmt. Figure 14A zeigt, dass je höher die Passage ist, umso kleiner wird die Mikromasse, was auf einer besseren Mikromassebildung beruht. In Figure 14B ist ersichtlich, dass die Mikromasse gebildet aus Passage 5 Zellen eine wesentlich rundere und besser ausgebildete Mikromasse formt, während die Mikromasse aus Passage 1 Zellen nach 4 Wochen eher noch aus Cluster besteht und keine charakteristische dichtere Außenschicht Richtung Medium ausbildet. Das optische Erscheinungsbild der Mikromasse gibt bereits wesentliche Informationen über den Zustand der Zellen.

### Beispiel 3: Größenabnahme als Indikator für die Redifferenzierung von Chondrozyten

### Methoden

### Chipherstellung

Zur Reinigung der Grundlage des Chips, ein Objektträger (76 x 26 mm) von VWR, wurde zuerst in eine 2% Hellmanex® III Lösung gegeben und 5 Minuten ins Ultraschallbad behandelt. Anschließend wurde die Lösung verworfen und der Vorgang zuerst mit Isopropanol und anschließend mit Wasser wiederholt. Zuletzt wurden die Objektträger mit Druckluft und 1 Stunde bei 80 °C im Ofen getrocknet.

Für die Chipherstellung, mussten zuerst 5 PDMS Schichten (76 x 26 mm) ausgeschnitten werden. Dafür wurde 0.5 mm dicke PDMS Folie in den Schneider CAMM-1 GS-24 von Roland DG eingefügt und die gewünschte Form ausgeschnitten, welche mit einer Pinzette entfernt wurden. Dann wurden die einzelnen Schichten durch Plasmaaktivierung miteinander verbunden, beginnend mit dem Mikroskopslide und der ersten Schicht PDMS Folie. Die Stärke der Verbindung wurde durch Hitzebehandlung im Ofen (80 °C) verstärkt.

Vor der Verwendung des Chips wurden alle Kammern, Kanäle und Reservoirs mit 70% Ethanol gespült. Die Zellkammern wurden zudem mit Lipidure®, einem Antifouling-Mittel, beschichtet. Zuletzt wurde der Chip mit UV-Licht sterilisiert.

### Zellkultur

Chondrozyten, gekauft von Sigma Aldrich (C-12710), wurden in Kulturflaschen mit Chondrozyten Wachstumsmedium von Sigma Aldrich (411-500) kultiviert (37 °C, 5% CO2). Bei 90% Konfluenz wurden die Zellen aus der Flasche gelöst. Dafür wurden die Zellen 5 Minuten mit 1X Trypsinlösung bei 37 °C inkubiert und anschließend Medium hinzugefügt, um die Reaktion zu stoppen. Die Zellsuspension wurde zentrifugiert (4 °C, 1400 rpm, 5 min) und eine Probe davon währenddessen mit Trypanblau gezählt. Das Zellpellet wurde in Medium resuspendiert und die benötigte Menge an Zellen für die Chipkulturen abgetrennt. Nach erneutem Zentrifugieren wurde das Zellpellet vorsichtig mit Fibrin vermischt (2000 Zellen pro µl Fibrin) und mit jeweils 30 µl aliquotiert. Zum Befüllen einer Zellkammer wurde 30 µl Thrombin (4 U/ml) mit Fibrin vermischt und sofort in den Chip geladen. Der Chip wurde 15 Minuten bei 37 °C inkubiert, damit sich ein festes 3D Netzwerk formt. Anschließend wurde Chip Kultivierungsmedium (DMEM, 1% Antibiotikum, 1% MEM NEAA, 1% ITS, 31.6 µg/ml L-Ascorbic acid 2-phosphate, 2% HEPES) mit unterschiedlichen Konzentrationen FBS und TGF-β3 oder Chondrozyten Differenzierungsmedium von Sigma Aldrich (411D-250) hinzugefügt. Die Chips wurden bis zu 4 Wochen bei 37 °C und 5% CO2 kultiviert, wobei jeden zweiten Tag Medium getauscht wurde.

### Resultate

Für eine Ko-Kultur von Chondrozyten und synovialen Fibroblasten sollte ein Medium gefunden werden, welches für beide Zellkulturen am Chip eingesetzt werden kann. Das Medium ausgehend von einer synovialen Fibroblasten Kultur am Chip wurde dahingehend geändert, dass die Serumkonzentration variiert wurde (1 und 10%) und TGF-β3, welches für die Redifferenzierung von Chondrozyten notwendig ist, hinzugefügt wurde. Diese Medien wurden mit einem gekauften Differenzierungsmedium verglichen. Figure 15 zeigt, dass die Größe des Chondrozytenklumpens wesentlich stärker über einen Zeitrahmen von 21 Tagen bei den selbst definierten Medien abnimmt. Während beim gekauften Differenzierungsmedium der Klumpen nach 21 Tagen noch größer als 50% das ursprünglich geladene Konstrukt ist. Es ist ersichtlich, dass mit einer geringeren Serumkonzentration anfangs die Abnahme geringer ist, doch nach 21 Tagen die Größe jenen, welche mit einer höheren Serumkonzentration kultiviert wurden, gleicht.

## Patentansprüche

1. Mikrofluidische Vorrichtung (1), vorzugsweise zum Herstellen einer dreidimensionalen Zellkultur,
mit mindestens einer Kammer (2) und einem die Kammer (2) zumindest teilweise durchlaufenden Fluidkanal (3) zum Bereitstellen eines die Kammer (2) vorzugsweise kontinuierlich durchlaufenden Fluidstroms, wobei die Kammer (2) mit einer Ladungsöffnung (4) verbunden und über die Ladungsöffnung (4) bis zu einem Sollfüllstand mit Hydrogel beladbar ist,
**dadurch gekennzeichnet, dass**
die Kammer (2) eine Hauptkammer (5) und eine mit der Hauptkammer (5) verbundene Nebenkammer (6) umfasst, wobei die Nebenkammer (6) beim Beladen der Kammer (2) mit Hydrogel bis zum Sollfüllstand zumindest teilweise mit Hydrogel befüllbar ist.

2. Vorrichtung (1) gemäß Anspruch 1, **dadurch gekennzeichnet, dass** die Hauptkammer (5) und die Nebenkammer (6) durch einen Verbindungskanal (7) verbunden sind, wobei der Verbindungskanal (7) einen kleineren Querschnitt als die Hauptkammer (5) und die Nebenkammer (6) aufweist.

3. Vorrichtung (1) gemäß einem der Ansprüche 1 oder 2, **dadurch gekennzeichnet, dass** der Fluidkanal (3) die Hauptkammer (5) zumindest teilweise durchläuft, und die Hauptkammer (5) einen ersten Kammerboden (8) aufweist, wobei der Fluidkanal (3) einen, beabstandet von dem ersten Kammerboden (8) in der Hauptkammer (5) angeordneten Fluideinlass (9), sowie einen beabstandet von dem ersten Kammerboden (8) in der Hauptkammer (5) angeordneten Fluidauslass (10) umfasst.

4. Vorrichtung (1) gemäß einem der Ansprüche 1 bis 3, **dadurch gekennzeichnet, dass** die Hauptkammer (5) einen ersten Kammerboden (8) aufweist und die Nebenkammer (6) einen zweiten Kammerboden (11) aufweist, wobei der erste Kammerboden (8) auf einem niedrigeren Höhenniveau als der zweite Kammerboden (11) ausgebildet ist.

5. Vorrichtung (1) gemäß Anspruch 3 oder 4, **dadurch gekennzeichnet, dass** der Fluidkanal (3) einen Fluidkanalboden (16) aufweist, wobei der Fluidkanalboden (16) auf einem höheren Höhenniveau als der erste Kammerboden (8) ausgebildet ist.

6. Vorrichtung (1) gemäß Anspruch 5, **dadurch gekennzeichnet, dass** der Fluidkanalboden (16) auf einem niedrigeren Höhenniveau als der zweite Kammerboden (11) ausgebildet ist.

7. Vorrichtung (1) gemäß einem der Ansprüche 1 bis 6, **dadurch gekennzeichnet, dass** die Nebenkammer (6) ein kleineres Volumen als die Hauptkammer (5) aufweist.

8. Vorrichtung (1) gemäß einem der Ansprüche 1 bis 7, **dadurch gekennzeichnet, dass** die Ladungsöffnung (4) mit der Nebenkammer (6) verbunden ist.

9. Vorrichtung (1) gemäß einem der Ansprüche 1 bis 8, **dadurch gekennzeichnet, dass** die Nebenkammer (6) an einer, dem Fluidkanal (3) gegenüberliegenden Seite (12) der Hauptkammer (5) mit der Hauptkammer (5) verbunden ist.

10. Vorrichtung (1) gemäß einem der Ansprüche 1 bis 9, **dadurch gekennzeichnet, dass** der Fluidkanal (3) entlang einer Seitenwand (13) der Hauptkammer (5) verläuft.

11. Vorrichtung (1) gemäß einem der Ansprüche 1 bis 10, **dadurch gekennzeichnet, dass** die Vorrichtung (1) eine die Ladungsöffnung (4) mit der Kammer (2) verbindende Leitung (15) umfasst.

12. Vorrichtung (1) gemäß einem der Ansprüche 1 bis 11, **dadurch gekennzeichnet, dass** die Kammer (2), die Hauptkammer (5), die Nebenkammer (6) und/oder der Fluidkanal (3) eine anwuchsverhindernde Oberflächenbeschichtung umfasst, wobei diese vorzugsweise ausgewählt ist aus der Gruppe bestehend aus einem Polyethylenglykol (PEG)-basierendem Polymer, einem Polysaccharid, insbesondere Agarose, einem Polyhydroxypolymer, insbesondere Poly(2-Hydroxyethylmethacrylat) (poly-HEMA), 2-Methacryloyloxtethylphosphorylcholin (MPC), Dextran oder Hydroxyethylcellulose (HEC), einem natürlichen Polymer, insbesondere einem S-Schichtprotein und Kombinationen davon.

13. Träger (17) umfassend mindestens eine mikrofluidische Vorrichtung gemäß einem der Ansprüche 1 bis 12.

14. Verfahren zum Herstellen einer dreidimensionalen Zellkultur oder eines Organoids mit einer mikrofluidischen Vorrichtung (1) gemäß einem der Ansprüche 1 bis 12, umfassend einen Schritt (A) des Beladens einer Kammer (2) der Vorrichtung (1) durch eine Ladungsöffnung (4) mit Hydrogel bis zu einem Sollfüllstand.

15. Verfahren gemäß Anspruch 14, **dadurch gekennzeichnet, dass** Zellen gemeinsam in und/oder vor Schritt (A) durch eine Ladungsöffnung (4) und/oder über einen Fluideinlass (9) in die Kammer (2) eingebracht werden, wobei die Zellen vorzugsweise ausgewählt sind aus Stammzellen und primären Zellen, vorzugsweise Fibroblasten, Knorpelzellen, Endothelzellen, Epithelzellen, Fettzellen, induziert pluripotente Stammzellen (IPS), Osteoclasten, Osteoblasten oder Osteozyten.

16. Verfahren zur Bestimmung des Einflusses einer chemischen Verbindung und/oder mindestens eines physikalischen Parameters auf Zellen einer dreidimensionalen Zellkultur in einer mikrofluidischen Vorrichtung gemäß einem der Ansprüche 1 bis 12 umfassend die Schritte:
a) Feststellen eines Erstzustandes der Zellen,
b1) Inkontaktbringen der Zellen mit einer chemischen Verbindung und/oder
b2) Änderung eines physikalischen Parameters innerhalb der mikrofluidischen Vorrichtung,
c) Feststellen eines Zweitzustandes der Zellen und
d) Bestimmen des Einflusses der chemischen Verbindung und/oder des mindestens einen physikalischen Parameters auf die Zellen durch Ermittlung einer Differenz zwischen dem Erstzustand und Zweitzustand der Zellen.
